# EUROPEAN PATENT APPLICATION

(11) **EP 2 233 075 A1**
(43) Date of publication of application: **29.09.2010**
(21) Application number: 10157123.0
(22) Date of filing: 19.03.2010
(51) Int. Cl.: A61B 5/1455

(54) **Method and apparatus for determining an indicator of autonomic nervous system state**

(30) Priority: 26.03.2009 US 411775
(71) Applicant: GENERAL ELECTRIC COMPANY, Schenectady, NY 12345 (US)
(72) Inventor: Paloheimo, Markku, 02320, Espoo (FI)
(74) Representative: Illingworth-Law, William Illingworth

(57) **Abstract**

A method and apparatus for determining an indicator of the level of nociception of a subject are disclosed. A parameter indicative of sympathetical activation in a subject is generated. To tackle inter-subject variability and to maintain low measurement delay with the help of reduced computational effort, changes in the parameter are monitored (41) to detect a stable state of the parameter. Upon detection the stable state, a subject-specific scaling transformation intended to transform the parameter to an index on a predetermined index scale is determined (42). The scaling transformation is made dependent on at least one value detected for the parameter in connection with detection of the stable state. The scaling transformation is then applied (44) to subsequent values of the parameter, thereby to transform the subsequent values to index values indicative of the level of nociception.

## Description

### Background of the Invention

This disclosure relates to the determination of an indicator of the state of the autonomic nervous system (ANS) of a subject, especially to the determination of an indicator indicative of the level of nociception or antinociception of the subject.

Antinociception normally refers to the blocking or suppression of nociception in the pain pathways at the subcortical level. It may be described as subcortical analgesia, in distinction to preventing the perception of pain at the cortex, i.e. cortical analgesia.

The autonomic nervous system is the 'unconscious' nervous system, which controls and regulates virtually all of our basic body functions, such as cardiac function, blood circulation and glandural secretion. The main parts of the ANS are the parasympathetic and sympathetic nervous branches. The sympathetic nervous system usually prepares us for high stress situations by speeding up the body functions. Under conditions of normal ANS regulation, the parasympathetic system restores the normal conditions in blood circulation by slowing down the heart rate, while conscious and even unconscious pain, discomfort, and surgical stress activate the sympathetical branch of the ANS and cause an increase in blood pressure, heart rate and adrenal secretions.

During the past few years, several commercial devices for measuring the level of consciousness and/or awareness in a clinical set-up during anesthesia have become available. The need for reliable monitoring of the adequacy of anesthesia is based on the quality of subject care and on economy related aspects. Balanced anesthesia reduces surgical stress and there is firm evidence that adequate analgesia decreases postoperative morbidity. Awareness during surgery with insufficient analgesia may lead to a post-traumatic stress disorder. Prolonged surgical stress sensitizes the central pain pathways, which increases pain sensations and secretion of stress hormones post-operatively. Low quality pre- and intra-operative analgesia makes it difficult to select the optimal pain management strategy later on. More specifically, it may cause exposure to unwanted side effects during the recovery from the surgery. Too light an anesthesia with insufficient hypnosis may cause traumatic experiences both for the subject and for the anesthesia personnel. From economical point of view, too deep an anesthesia may cause increased perioperative costs through extra use of drugs and time, and also extended time required for post-operative care. Too deep a sedation and/or hypnosis may also cause complications and prolong the usage time of expensive facilities, such as the intensive care theater.

Many prior art technologies that are claimed to measure the adequacy of analgesia show a considerable dependence on the level of hypnosis and, consequently, at light anesthesia without any noxious stimulations show a value that is usually associated with poor analgesia. A further drawback of the prior art technologies is that the measurement values show a considerable inter-subject variability, due to the variability in the associated physical signals/parameters between different subjects. It is therefore difficult to interpret the adequacy of anesthesia from the point of view of analgesia. This difficulty applies particularly to the verification of the level of nociception of a variety of subjects against a fixed scale.

In order to provide quantitative information of the level of nociception in sedated or anesthetized subjects, it has been suggested that the nociceptive state of a subject be determined based on one or more physiological signals or parameters that reflect the pain state of the subject by applying to said signal(s) or parameter(s) to a subject-adaptive normalization transform that adapts to the subject in question and scales the input values to a predetermined value range, thereby to obtain an index of nociception. For improved specificity, the index of nociception may be a composite indicator determined based on at least two physiological signals or parameters. Each signal or parameter is subjected to a normalization transform and the composite indicator is determined as a weighted average of the normalized values corresponding to each other in time domain. The normalization transform may be implemented by various techniques that include the use of a parameterized function comprising at least one subject-specif parameter and the use of a so-called histogram transform that may adapt to the incoming signal.

The above-mentioned normalization and averaging processes for generating subject-specific quantitative information of the level of nociception on a fixed scale require rather high computing power to keep the delay between the actual physiological response and the corresponding response in the index low. It is therefore desirable to obtain a solution in which the final index, in which the inter-subject variability has been taken into account, may be derived from an individual physiological signal or parameter in a less complex way, thereby to achieve low measurement delay with the help of reduced computing power.

### Brief Description of the Invention

The above-mentioned problem is addressed herein which will be comprehended from the following specification.

In an embodiment, a method for determining an indicator of the level of nociception of a subject comprises generating a parameter indicative of sympathetic state in a subject and monitoring changes in the parameter, thereby to detect a stable state of the parameter. The method further comprises defining, upon detecting the stable state, a subject-specific scaling transformation intended to transform the parameter to an index on a predetermined index scale, wherein the defining includes making the scaling transformation dependent on at least one value detected for the parameter in connection with detection of the stable state, and wherein the index serves as the indicator of the level of nociception. The method further includes applying the scaling transformation to subsequent values of the parameter, thereby to transform the subsequent values to index values indicative of the level of nociception.

In another embodiment, an apparatus for determining an indicator of the level of nociception of a subject comprises a parameter unit configured to generate a parameter indicative of sympathetic activation in a subject, a monitoring unit configured to monitor changes in the parameter, thereby to detect a stable state of the parameter, and a scaling unit configured to define a subject-specific scaling transformation intended to transform the parameter to an index on a predetermined index scale, wherein the scaling unit is configured to make the scaling transformation dependent on at least one value detected for the parameter in connection with detection of the stable state, and wherein the index serves as the indicator of the level of nociception. The apparatus further comprises an index determination unit configured to apply the scaling transformation to subsequent values of the parameter, thereby to transform the subsequent values to index values indicative of the level of nociception.

In a still further embodiment, a computer program product for determining an indicator of the level of nociception of a subject comprises a first program product portion configured to generate a parameter indicative of sympathetic activation in a subject, a second program product portion configured to monitor changes in the parameter, thereby to detect a stable state of the parameter, and a third program product portion configured to define a subject-specific scaling transformation intended to transform the parameter to an index on a predetermined index scale, wherein the third program product portion is configured to make the scaling transformation dependent on at least one value detected for the parameter in connection with detection of the stable state, and wherein the index serves as the indicator of the level of nociception. The computer program product further comprises a fourth program product portion configured to apply the scaling transformation to subsequent values of the parameter, thereby to transform the subsequent values to index values indicative of the level of nociception.

Various other features, objects, and advantages of the invention will be made apparent to those skilled in the art from the following detailed description and accompanying drawings.

### Brief Description of the Drawings

FIG. 1 illustrates four values of an ANS parameter determined based on a plethysmographic signal obtained from a subject;
FIG. 2 is a flow diagram illustrating the determination of the ANS parameter of FIG. l;
FIG. 3 illustrates the behavior of the ANS parameter of FIG. 1 during noxious stimuli;
FIG. 4 is a flow diagram illustrating the calibration carried out prior to the actual ANS state index measurement;
FIG. 5 illustrates the calibration and measurement phases in connection with the example of FIG. 3,
FIG. 6 illustrates one embodiment of a system for determining the ANS state index; and
FIG. 7 illustrates the operational entities of the control and processing unit of the system of FIG. 6.

### Detailed Description of the Invention

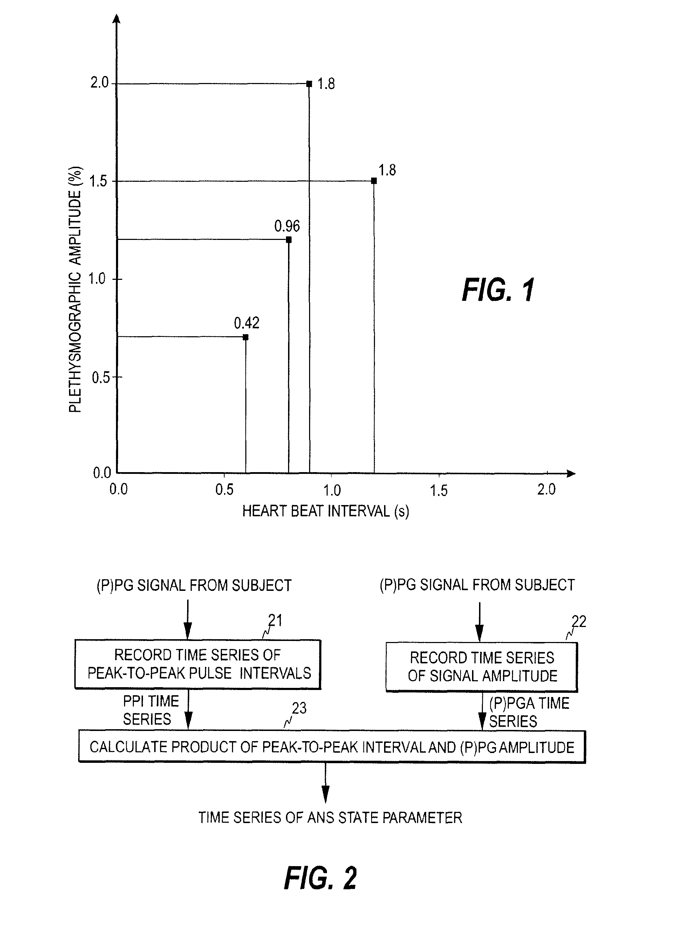

In the embodiment of FIG. 1, an ANS state parameter describing the state of the autonomic nervous system (ANS) is determined based on the product of two physiological parameters regulated by the ANS. It is assumed in this context that the said parameters represent the plethysmographic pulse amplitude (PGA) and the heart beat interval termed peak-to-peak pulse interval (PPI) in this context. On a rectangular coordinate system shown in FIG. 1, where the abscissa represents one of the parameters and the ordinate the other, the product of the two physiological parameters corresponds to an area whose magnitude is regulated by the operation of the ANS. Both the PGA and the PPI may be determined through a pulse oximeter from a finger of the subject based on one or more plethysmographic signals measured by the pulse oximeter. Sympathetic activation decreases peripheral arterial tone which in turn decreases the amplitude of the (photo)plethysmographic ((P)PG) signal measured by the pulse oximeter. Sympathetic activation also increases the heart rate, i.e. decreases the peak-to-peak pulse interval. Thus, the magnitude of the area is indicative of the level of sympathetic activation and thus also of the level of pain, discomfort, and surgical stress, i.e. the level of nociception or antinociception. As shown in FIG. 1, two products (areas) may be equal even though the corresponding physiological parameters contributing to each product are different. Pulse oximeter normally measures the percentage of the pulsating component (i.e. AC component) of light absorption of the non-pulsating (i.e. DC component) of light absorption.

FIG. 2 illustrates one embodiment of the determination of the ANS state parameter of FIG. 1. From a (photo)plethysmographic signal obtained from a subject the peak-to-peak pulse intervals are extracted at step 21, whereby a time series of PPI is obtained from step 21. Additionally, the pulse amplitude of the (photo)plethysmographic signal is extracted for each pulse beat at step 22, whereby a time series of the (P)PG amplitude, i.e. the amplitude of pulsative component of the peripheral blood circulation, is obtained from step 22. At step 23, the time series of the product of the peak-to-peak pulse interval and the (P)PG amplitude is calculated by multiplying the value of each interval with the value of the next (P)PG amplitude. As the product, i.e. the ANS state parameter, represents the sympathetic tonus of the ANS, the time series output from step 23 is indicative of sympathetical activations occurring in the ANS and thus also of pain and discomfort of the subject.

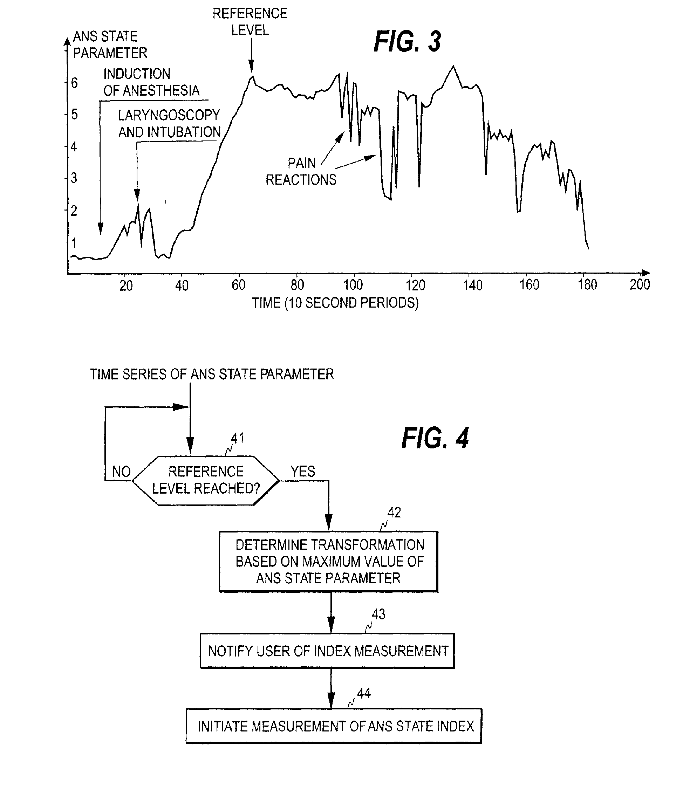

FIG. 3 illustrates the behavior of the ANS state parameter of FIG. 1 during surgery. After the induction of anesthesia and the initial procedures that cause sympathetic activation the subject calms down and the ANS state parameter increases until it reaches a certain level that is termed a reference level in this context. In the induction of anesthesia the sympathetic tonus decreases and the peripheral blood flow resistance decreases as the arterioles relax. As a result, the amplitude of the photoplethysmographic signal measured from a finger increases. However, this requires that the subject is normovolemic so that reduced blood volume does not impede the increase of the said amplitude. Normovolemia may be detected by measuring the temperature of middle finger tip. The maximum of the ANS state parameter, i.e. the reference level, is reached when the heart beat has also calmed down. When the reference level is reached, the ANS is in a stable state in which PGA is reactive to pain and pain is clearly reflected in the value of the ANS state parameter, as is shown in the figure. Near the end of the surgery, the effect of the anesthetics diminishes and therefore the ANS state parameter has a downward trend.

For the measurement of the final ANS state index the system tracks the changes in the ANS state parameter to detect when the parameter has reached the reference level. When this occurs, a subject-specific scaling transformation is determined. Based on the subject-specific scaling transformation the measurement of the ANS state index may then be initiated. The period from the start of the measurement of the ANS state parameter to the initiation of the actual ANS state index measurement is here termed a calibration period, since during that period the system is individually calibrated for the measurement of the ANS state index. Due to the calibration period, subsequent need of antinociceptive medication may be evaluated efficiently.

FIG. 4 illustrates one embodiment for carrying out calibration steps during the calibration period. At step 41, the system continuously performs a stability check in order to detect when the ANS state parameter has reached the reference level and thus also the stable state. The stability check may be carried out by calculating a measure that indicates the reduction of change or the reduction of variability that occurs in the ANS state parameter when it reaches the reference level. To detect the stable state, the said measure may be compared with a predetermined threshold. When the measure fulfills predetermined criteria with respect to the threshold, the stable state is detected. In one embodiment, a measure of variability or irregularity, such as the entropy of the ANS state parameter may be calculated for each pulse beat based on the time sequence of the ANS state parameter. As the entropy drops to a minimum when the ANS state parameter reaches the reference level, the stable state may be detected based on the behavior of the entropy of the ANS state parameter. Here, the value of the ANS state parameter may also be determined by calculating the average over a certain period, such as ten heart beats, or by using median filtering to remove noise. Other measures that may be used in the stability check are the variance, standard deviation, or coefficient of variation of the ANS state parameter, for example. The reduction of the said measure of variability or irregularity is thus utilized to detect the stable state. For example, the stable state may be detected when the said measure drops below a predetermined threshold or stays below a predetermined threshold for a predetermined period.

Normally, when the ANS state parameter starts to increase due to the effect of sedative or anaesthetic medication, both PPinterval and PGA, and their product (i.e. the ANS state parameter) grow steadily and, finally, the ANS state parameter stabilizes to a maximum value with certain minimum variability. Signals from subjects having arrhythmias, such as extrasystolia, atrial fibrillation altion, or a variety of conduction abnormalities should be subjected to median filtering procedures to detect the reference level of the ANS state parameter. If the pulse oximeter sensor is changed to another finger, the reference level of ANS state parameter has to be determined again, as in a typical case all fingers have slightly different maximum pulse amplitudes even if their temperatures are all above 32 °C (i.e. are indicative of normovolemia).

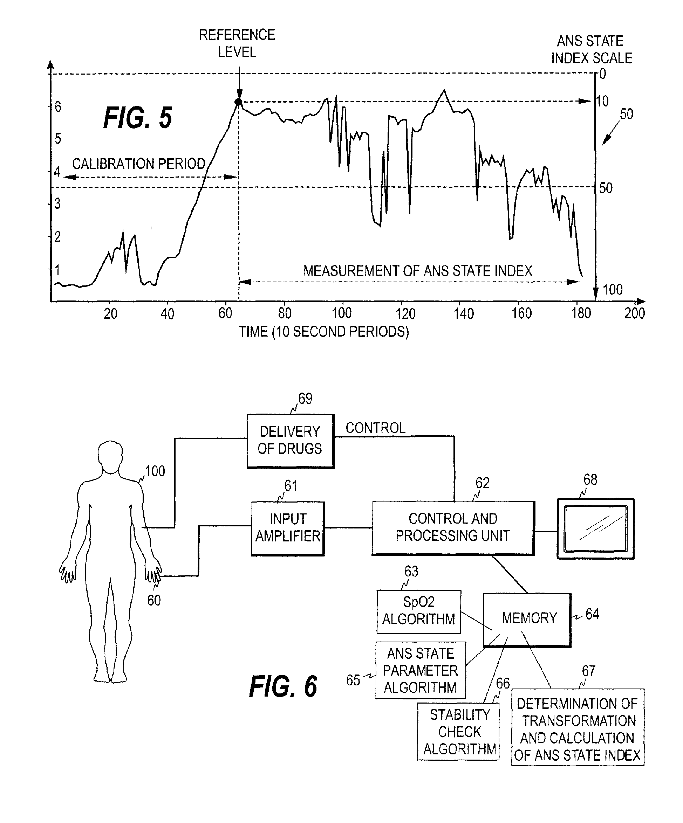

When the stability check 41 indicates that the ANS state parameter has reached the reference level, the maximum value of the ANS state parameter reached is stored and a subject-specific scaling transformation is determined based on the said value (step 42). The transformation is employed to map the ANS state parameter values to a predetermined index scale, thereby to obtain an ANS state index indicative of the level of nociception. For example, the said value of the ANS state parameter may be transformed to a value of ten and the (subsequent) values, which are lower than the maximum found, may be made to slide linearly between 10 and 100, as is illustrated with index scale 50 in FIG. 5 that shows the ANS state parameter behavior of FIG. 3. The black dot in FIG. 5 represents the maximum of the ANS state parameter that is found based on the stability check. Thus, in this example a subsequent ANS state index value may be found based on the equation ANSSI = 100 -(ANSSP/ANSSPm)×90, where ANSSI is the index value, ANSSPm is the found maximum value and ANSSP is a subsequent ANS state parameter value. Another formula that may be used for the calculation of the ANS state index is ANSSI = 10 + 90×[(ANSSPm-ANSSP)/ANSSPm].With reference to FIG. 4 again, the measurement of the ANS state index may begin after step 42 as the subject-specific scaling transformation is now available. At this stage, the user of the apparatus may be notified that the reference level has been found for the subject and the measurement of the index initiates (steps 43 and 44 in FIG. 4). The measurement may also be initiated only after an acknowledgment to the notification is received from the user. In the index measurement, the scaling transformation is applied to subsequent values of the ANS state parameter, thereby to transform the subsequent values to ANS state index values.

In the above-described manner the point (moment of time and ANS state parameter value) may be determined for each subject, after which the ANS state parameter remains stable until the subject senses pain, i.e. the subject-specific point may be determined after which the ANS state parameter has its full responsiveness to pain. The senses of pain lead to an increase in the ANS state index, as can be seen from FIGs. 3 and 5. Antinociceptive medication (opioids) may be used to reverse these changes to return the ANS state parameter to a stable state. Based on the index, a physician or a system may thus draw conclusions on whether the level of antinociception of the subject needs to be controlled. Such a system may be a drug delivery system or a decision support system for a physician, for example. Since the index is indicative, depending on the point of view, of the level of nociception or the level of antinociception, it is also indicative of the nociception/antinociception balance.

After the ANS state parameter has reached the stable state, the ANS state index may thus be determined in a very simple and straightforward manner based the product of the physiological signals/parameters, i.e. PGA and PPI, since the determination requires only a multiplication and a simple scaling operation. The scaling operation may also be a single multiplication by a gain factor if the ANS state index scale is reversed, i.e. if the stable state represents a high index value and if the index value drops in response to pain reactions. More generally, a gain factor may be used if the ANS state parameter and the ANS state index react, contrary to the example above, in the same direction in response to pain. The gain factor may also be determined based on the extreme parameter value detected in connection with the detection of the stable state. Instead of a maximum value, the extreme value may also be a minimum value, if the ANS state parameter is chosen so that it decreases towards the stable state. Furthermore, this subject-specific parameter value used as a parameter in the scaling transformation, such as ANSSPm in the above examples, or used to determine the gain factor, does not necessarily have to be the extreme parameter value (minimum or maximum) detected in connection with the detection of the stable state, but the next value after the detection of the stable state or a short-time average value calculated after the detection of the stable state may also be used, for example. The transformation may also be nonlinear. At any rate, in all embodiments the state of the subject may be verified against a fixed ANS state index scale, although the ANS state parameter values are subject-specific. The scaling transformation may also be re-determined in the above manner, should the ANS state parameter change so that the ANS state index value moves outside the scale.

FIG. 6 illustrates one embodiment of the system or apparatus for determining the ANS state index indicative of the level of (anti)nociception. As discussed above, the ANS state parameter may be determined based on a photoplethysmographic signal obtained from a probe 60 attached to a finger of a subject 100. The probe includes a light source for sending an optical signal through the tissue and a photodetector for receiving the signal transmitted through or reflected from the tissue. The light propagated through or reflected from the tissue is received by a photodetector, which converts the optical signal received at each wavelength into an electrical signal pulse train and feeds it to an input amplifier 61. The amplified signal is then supplied to a control and processing unit 62, which converts the signals into digitized format for each wavelength channel. The digitized signal data is then utilized by a conventional SpO₂ algorithm 63 adapted to record the time series of the photoplethysmographic signal amplitude in a memory 64 of the control and processing unit.

The control and processing unit is further provided with an ANS state parameter algorithm 65 adapted to determine, when executed by the control and processing unit, the time sequence of the ANS state parameter. As shown in FIG. 2, this includes the determination of the peak-to-peak pulse interval based on the photoplethysmographic signal and the recording the time series thereof. The control and processing unit further includes a stability check algorithm 66. When executed by the control and processing unit, the stability check algorithm monitors the ANS state parameter to detect when the ANS state parameter reaches the reference level and thus also a stable state. As discussed above, this may involve calculating the entropy of the ANS state and comparing the entropy value with a predetermined threshold. When the entropy drops below the threshold, the algorithm decides that the ANS state parameter has reached a stable state and the subject-specific scaling transformation may be determined. The control and processing unit is thus also provided with an algorithm 67 for determining and using the subject-specific scaling transformation in the above-described manner.

During the actual measurement, i.e. after the calibration period, the control and processing unit thus employs the determined scaling transformation to transform the ANS state parameter values to ANS state index values. As discussed above, the control and processing unit may also, if necessary, re-determine the transformation during the stable state.

The control and processing unit may display the results, such as the index values, through at least one monitor 68 and/or it may further supply the ANS state index as input data to a device or system 69 configured to deliver antinociceptive drugs to the subject, thereby to enable automatic control of the level of nociception of the subject. It is thus also possible, that the ANS state index is used as the input data only, without displaying it to the user. The control and processing unit may act as a controlling entity controlling the administration of the drugs from the delivery system to the subject. Alternatively, the control and processing unit may supply the ANS state index to another computer unit or microprocessor (not shown), which then acts as the controlling entity controlling the drug delivery system. The said controlling entity is provided with the control data needed for the administration, such as the pharmacodynamic and pharmacokinetic properties of the drugs to be administered. The drug delivery system may comprise separate delivery units for one or more drugs to be administered, e.g. hypnotics/anesthetic gas, and opioids. The monitor 68 may also be part of a decision support system for a physician.

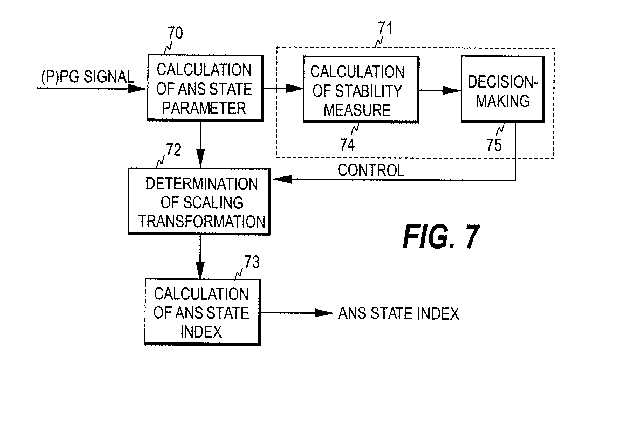

The control and processing unit, which is adapted to execute the above-described algorithms, may thus be seen as an entity of four operational modules or units, as is illustrated in FIG. 7: a parameter determination unit 70 configured to determine the time series of the ANS state parameter, a monitoring unit 71 configured to monitor and possibly filter changes in the ANS state parameter and to detect when the ANS state parameter reaches the stable state, a scaling unit 72 configured to determine the scaling transformation when the monitoring unit indicates that the ANS state parameter has reached the stable state, and an index determination unit 73 configured to determine the time series of the ANS state index based on the time series of the ANS state parameter and the scaling transformation. The monitoring unit includes a calculation sub-unit 74 configured to calculate a stability/irregularity measure for the ANS state parameter, such as the entropy of the ANS state parameter, and a decision-making sub-unit 75 configured to make the decision on the stable state and the initiation of the index measurement.

A conventional pulse oximeter device may be upgraded to enable the device to determine the ANS state index in the above-described manner based on the signal data that the device measures from the subject. Such an upgrade may be implemented, for example, by delivering to the device a software module that enables the device to determine the ANS state index based on the plethysmographic data measured by the device, i.e. a module including elements 65-67 of FIG. 6. The software module may be delivered, for example, on a data carrier, such as a CD or a memory card, or the through a telecommunications network. A separate index measurement device, provided with a control and processing unit; a memory storing the above algorithms; and a display unit, may also be connected to a conventional pulse oximeter for utilizing the plethysmographic signal data in the above manner. This device is thus provided with access to the plethysmographic signal data measured by the pulse oximeter.

Above, the product of plethysmographic amplitude and the heart beat interval is used as the ANS state parameter. However, the ANS state parameter is not limited to these parameters but different signals/parameters may be used. Instead of PGA, a signal indicative of pulse pressure or a signal indicative of arterial pressure may also be used, although is not verified. The heart beat interval may also be derived from various other physiological signals, e.g. electrocardiography. However, the plethysmograhic signal is beneficial in the sense that only a finger probe is needed to measure the ANS state parameter. Instead of the product, an appropriate linear or non-linear combination of the signals/parameters may also be used, such as a weighted or an unweighted average of the signals/parameters. Provided that the product is used, a prerequisite is that the physiological signals/parameters contributing to the product change in the same direction in response to a change in sympathetic activation of the subject, so that the response to sympathetic activation remains unambiguous.

Although the above examples use a surgery as an example of an application environment, the index determination mechanism is also suitable for sedated subjects in intensive care or during endoscopic examinations, for example.

This written description uses examples to disclose the invention, including the best mode, and also to enable any person skilled in the art to make and use the invention. The patentable scope of the invention is defmed by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.
1. A method for determining an indicator of the level of nociception of a subject, the method comprising:
   generating a parameter indicative of sympathetic state in a subject;
   monitoring changes in the parameter, thereby to detect a stable state of the parameter;
   upon detecting the stable state, defining a subject-specific scaling transformation intended to transform the parameter to an index on a predetermined index scale, wherein the defining includes making the scaling transformation dependent on at least one value detected for the parameter in connection with detection of the stable state, and wherein the index serves as the indicator of the level of nociception; and
   applying the scaling transformation to subsequent values of the parameter, thereby to transform the subsequent values to index values indicative of the level of nociception.
2. The method according to clause 1, wherein the generating includes generating a product of a first physiological signal and a second physiological signal.
3. The method according to clause 1 or clause 2, wherein the generating includes generating the product, in which the first physiological signal comprises a time series of heart beat intervals of the subject and the second physiological signal a time series of pulse amplitudes of a plethysmographic signal obtained from the subject, and wherein the generating further includes determining a time series of products in which each product represents the product of one of the heart beat intervals and a pulse amplitude that is the next pulse amplitude, after said one of the heart beat intervals, in the time series of pulse amplitudes.
4. The method according to anyone of the preceding clauses, wherein the monitoring includes determining one of a measure of irregularity and a measure of variability of the parameter.
5. The method according to clause 4, wherein the monitoring further includes comparing the measure with a predetermined limit value and detecting the stable state when the measure fulfills a predetermined criterion with respect to the predetermined limit value.
6. The method according to clause 4 or clause 5, wherein the determining the measure includes determining entropy of the parameter.
7. The method according to any one of the preceding clauses, wherein the defining includes making the scaling transformation dependent on the at least one value of the parameter, in which the at least one value is an extreme value detected for the parameter in connection with the detection of the stable state.
8. The method according to any one of the preceding clauses, wherein the defining includes defining the scaling transformation, in which the scaling transformation is a linear transformation.
9. An apparatus for determining an indicator of the level of nociception of a subject, the apparatus comprising:
   a parameter unit configured to generate a parameter indicative of sympathetic activation in a subject;
   a monitoring unit configured to monitor changes in the parameter, thereby to detect a stable state of the parameter;
   a scaling unit configured to define a subject-specific scaling transformation intended to transform the parameter to an index on a predetermined index scale, wherein the scaling unit is configured to make the scaling transformation dependent on at least one value detected for the parameter in connection with detection of the stable state, and wherein the index serves as the indicator of the level of nociception;
   an index determination unit configured to apply the scaling transformation to subsequent values of the parameter, thereby to transform the subsequent values to index values indicative of the level of nociception.
10. The apparatus according to clause 9, wherein the parameter unit is configured to generate the parameter as a product of a first physiological signal and a second physiological signal.
11. The apparatus according to clause 9 or clause 10, wherein the first physiological signal represents heart beat interval of the subject and the second physiological signal represents amplitude of a plethysmographic signal obtained from the subject.
12. The apparatus according to any one of clauses 9 to 11, wherein the monitoring unit is configured to determine one of a measure of irregularity and a measure of variability of the parameter.
13. The apparatus according to clause 12, wherein the monitoring unit is further configured to compare the measure with a predetermined limit value and to detect the stable state when the measure fulfills a predetermined criterion with respect to the limit value.
14. The apparatus according to clause 12 or clause 13, wherein the monitoring unit is configured to determine the measure of irregularity, in which the measure of irregularity represents the entropy of the parameter.
15. The apparatus according to any one of clauses 9 to 14, wherein the at least one value is an extreme value detected for the parameter in connection with the detection of the stable state.
16. The apparatus according to any one of clauses 9 to 15, wherein the scaling transformation is a linear transformation.
17. A computer program product for determining an indicator of the level of nociception of a subject, the computer program product comprising:
   a first program product portion configured to generate a parameter indicative of sympathetic activation in a subject;
   a second program product portion configured to monitor changes in the parameter, thereby to detect a stable state of the parameter; and
   a third program product portion configured to define a subject-specific scaling transformation intended to transform the parameter to an index on a predetermined index scale, wherein the third program product portion is configured to make the scaling transformation dependent on at least one value detected for the parameter in connection with detection of the stable state, and wherein the index serves as the indicator of the level of nociception; and
   a fourth program product portion configured to apply the scaling transformation to subsequent values of the parameter, thereby to transform the subsequent values to index values indicative of the level of nociception.

## Claims

1. A method for determining an indicator of the level of nociception of a subject (100), the method comprising:
generating (21-23) a parameter indicative of sympathetic state in a subject (100);
monitoring (41) changes in the parameter, thereby to detect a stable state of the parameter;
upon detecting the stable state, defming (42) a subject-specific scaling transformation intended to transform the parameter to an index on a predetermined index scale (50), wherein the defining includes making the scaling transformation dependent on at least one value detected for the parameter in connection with detection of the stable state, and wherein the index serves as the indicator of the level of nociception; and
applying (44) the scaling transformation to subsequent values of the parameter, thereby to transform the subsequent values to index values indicative of the level of nociception.

2. The method according to claim 1, wherein the generating includes generating (23) a product of a first physiological signal and a second physiological signal.

3. The method according to claim 2, wherein the generating (23) includes generating the product, in which the first physiological signal comprises a time series of heart beat intervals of the subject and the second physiological signal a time series of pulse amplitudes of a plethysmographic signal obtained from the subject, and wherein the generating further includes determining a time series of products in which each product represents the product of one of the heart beat intervals and a pulse amplitude that is the next pulse amplitude, after said one of the heart beat intervals, in the time series of pulse amplitudes.

4. The method according to any one of the preceding claims, wherein the monitoring (41) includes determining one of a measure of irregularity and a measure of variability of the parameter.

5. The method according to claim 4, wherein the monitoring further includes comparing the measure with a predetermined limit value and detecting the stable state when the measure fulfills a predetermined criterion with respect to the predetermined limit value.

6. The method according to any one of the preceding claims, wherein the defining (42) includes making the scaling transformation dependent on the at least one value of the parameter, in which the at least one value is an extreme value detected for the parameter in connection with the detection of the stable state.

7. The method according to any one of the preceding claims, wherein the defining (42) includes defining the scaling transformation, in which the scaling transformation is a linear transformation.

8. An apparatus for determining an indicator of the level of nociception of a subject (100), the apparatus comprising:
a parameter unit (62; 70) configured to generate a parameter indicative of sympathetic activation in a subject;
a monitoring unit (62; 71) configured to monitor changes in the parameter, thereby to detect a stable state of the parameter;
a scaling unit (62; 72) configured to define a subject-specific scaling transformation intended to transform the parameter to an index on a predetermined index scale (50), wherein the scaling unit is configured to make the scaling transformation dependent on at least one value detected for the parameter in connection with detection of the stable state, and wherein the index serves as the indicator of the level of nociception;
an index determination unit (62; 73) configured to apply the scaling transformation to subsequent values of the parameter, thereby to transform the subsequent values to index values indicative of the level of nociception.

9. The apparatus according to claim 8, wherein the parameter unit (62; 70) is configured to generate the parameter as a product of a first physiological signal and a second physiological signal.

10. The apparatus according to claim 8 or claim 9, wherein the first physiological signal represents heart beat interval of the subject and the second physiological signal represents amplitude of a plethysmographic signal obtained from the subject.

11. The apparatus according to any one of claims 8 to 10, wherein the monitoring unit (62; 71) is configured to determine one of a measure of irregularity and a measure of variability of the parameter.

12. The apparatus according to claim 11, wherein the monitoring unit (62; 71) is configured to determine the measure of irregularity, in which the measure of irregularity represents the entropy of the parameter.

13. The apparatus according to any one of claims 8 to 12, wherein the at least one value is an extreme value detected for the parameter in connection with the detection of the stable state.

14. The apparatus according to any one of claims 8 to 13, wherein the scaling transformation is a linear transformation.

15. A computer program product for determining an indicator of the level of nociception of a subject (100), the computer program product comprising:
a first program product portion (65) configured to generate a parameter indicative of sympathetic activation in a subject;
a second program product portion (66) configured to monitor changes in the parameter, thereby to detect a stable state of the parameter; and
a third program product portion (67) configured to define a subject-specific scaling transformation intended to transform the parameter to an index on a predetermined index scale, wherein the third program product portion is configured to make the scaling transformation dependent on at least one value detected for the parameter in connection with detection of the stable state, and wherein the index serves as the indicator of the level of nociception; and
a fourth program product portion (67) configured to apply the scaling transformation to subsequent values of the parameter, thereby to transform the subsequent values to index values indicative of the level of nociception.
